# EUROPEAN PATENT SPECIFICATION

(11) **EP 1 906 845 B1**
(45) Date of publication and mention of the grant of the patent: **28.10.2009**
(21) Application number: 06762460.1
(22) Date of filing: 06.07.2006
(51) Int. Cl.: A61B 17/17, A61G 13/04, A61B 17/16

(54) **SYSTEM FOR ALIGNING A MATERIAL-ABRADING TOOL RELATIVE TO AN INTERVERTEBRAL-DISC COMPARTMENT**
SYSTEM ZUR AUSRICHTUNG EINES MATERIAL-ABSCHEUERNDEN WERKZEUGS RELATIV ZU EINEM BANDSCHEIBEN-KOMPARTIMENT
SYSTÈME POUR ALIGNER UN OUTIL D ABRASION DE MATÉRIAU PAR RAPPORT À UN LOGEMENT DE DISQUE INTERVERTÉBRAL

(30) Priority: 06.07.2005 US 696887 P
(43) Date of publication of application: 09.04.2008
(73) Proprietor: Copf jun., Franz, 70184 Stuttgart (DE)
(72) Inventor: Copf jun., Franz, 70184 Stuttgart (DE)
(74) Representative: Schwanhäusser, Gernot
(86) International application number: PCT/EP2006/006607
(87) International publication number: WO 2007/003436

(56) References cited:
- EP-A- 1 442 715
- WO-A-01/97680

## Description

### BACKGROUND OF THE INVENTION

### 1. Field of the Invention

The invention relates to a system for aligning a material-abrading tool according to the preamble of claim 1.

A system according to the preamble of claim 1 is known from WO 01/97680 A.

### 2. Description of the State of the Art

From US 2002/0161446 A1 a device is known in which a material-abrading tool is guided on a reference frame. The reference frame is fastened laterally to an operating table and extends over and beyond the patient. The intervertebral-disc compartment is firstly measured out with the aid of imaging methods, with the vertical (i.e. the direction of the gravity force) being a reference direction. For this purpose the intervertebral-disc compartment is X-rayed from the side, so that the lordosis of the vertebral column relative to the vertical direction can be surveyed.

With the aid of a plumb bob or similar, the reference frame is aligned above the stationary operating table.

The vertical consequently represents the common reference system for the surveying of the intervertebral-disc compartment, on the one hand, and for the alignment of the reference frame, on the other hand.

Document WO 01/97680 A mentioned at the outset discloses a system comprising a frame supporting a holder for a surgical instrument. The frame can be rotated around a rotational axis that runs perpendicular to a longitudinal axis of an operating table to which the frame is mounted. Under medical imaging it is possible to rotate the frame such that an operating plane of the surgical instrument tool can be adjusted to the lordosis of a patient.

However, it turned out that with the use of such reference frames, even after intervertebral-disc operations during which no complications occurred, complaints occur time and again that are to be ascribed to incorrect loadings of the vertebral column.

### SUMMARY OF THE INVENTION

For this reason, it is an object of the present invention to provide a system for aligning a material-abrading tool relative to an intervertebral-disc compartment, with which the risk of complaints of such a type is reduced.

According to the invention, this object is achieved by a system according to claim 1.

The inventor has noted that the vertebral column is frequently not only curved towards the front or towards the rear, but may also be twisted in itself along its longitudinal direction. Such a twisting may have pathological causes, but it may also arise in the course of the exposing of a ventral access if tissue connected to the vertebrae is displaced to one side. If such a twisting of individual vertebrae or of several vertebrae is left out of account, the material-abrading tool will be set incorrectly, so that the intervertebral-disc prosthesis is also later received in the intervertebral-disc compartment in a position that is twisted about the longitudinal axis of the adjacent vertebrae in relation to the nominal position.

This fact is taken into account by virtue of the fact that the reference frame is not aligned parallel to the surface of the operating table but is aligned parallel to the vertebral plane of the vertebrae adjacent the intervertebral-disc compartment. The alignment is carried out based on the laterally recorded images. The images or, what is equivalent, the image-recording device thus constitute(s) the common reference system both for the alignment of the vertebral column by tilting of the operating table and for the alignment of the reference frame. In order to orient the intervertebral-disc compartment in relation to this reference system, the operating table is, for example, tilted in such a way that the two transverse processes of a vertebra delimiting the intervertebral-disc compartment substantially overlap one another in the images recorded from the side.

An alignment of the reference frame in the images is possible, for example, if the reference frame is rigidly connected to an alignment element that is detected by the images. The reference frame is then tilted about an axis parallel to the longitudinal axis of the operating table until the alignment element appears on the recorded images in the form of a predetermined pattern.

### BRIEF DESCRIPTION OF THE DRAWINGS

Various features and advantages of the present invention may be more readily understood with reference to the following detailed description taken in conjunction with the accompanying drawing in which:
- FIG. 1: in a greatly schematised front view of a system according to the invention prior to the tilt- ing of the operating table;
- FIG. 2: the system shown in FIG. 1 after the tilting of the operating table;
- FIG. 3: the system shown in FIG. 1 after the aligning of the reference frame in relation to a fluoro- scope;
- FIG. 4: a simplified perspective representation of a part of an alignment element;
- FIG. 5: a longitudinal section through the part shown in FIG. 4;
- FIG. 6a: an image recorded by the fluoroscope of the alignment element shown in FIGS. 4 and 5 if said element is aligned with the direction of propagation of the X-rays;
- FIG. 6b: the image recorded by the fluoroscope of the alignment element shown in FIGS. 4 and 5 if said element is not aligned with the direc- tion of propagation of the X-rays.

### DESCRIPTION OF PREFERRED EXEMPLARY EMBODIMENTS

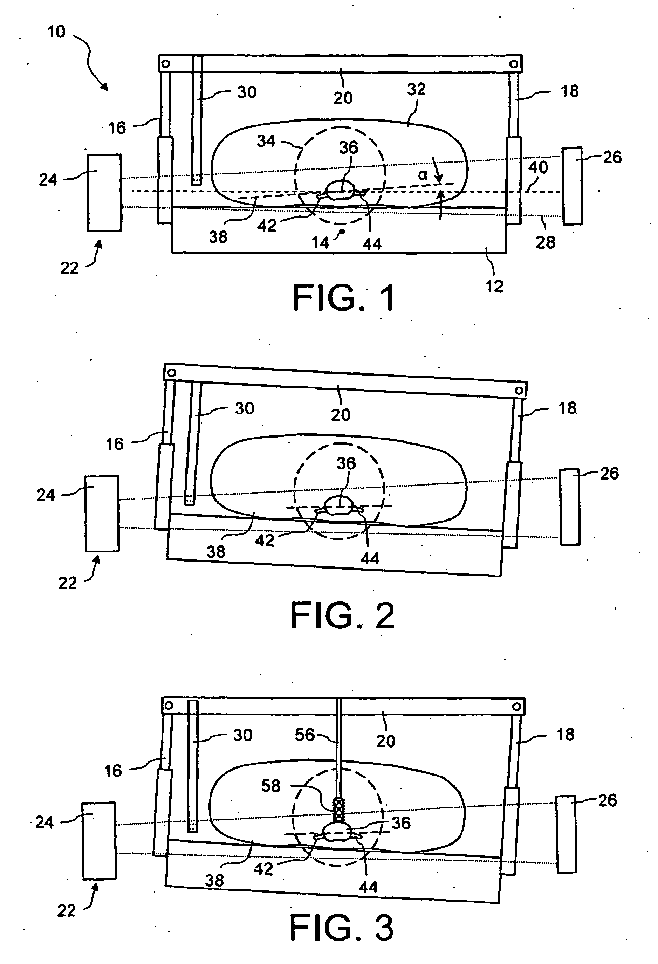

FIG. 1 shows, in a considerably schematised front view, a system 10 for aligning a material-abrading tool relative to an intervertebral-disc compartment in a configuration prior to the start of an intervertebral-disc operation.

The system 10 comprises an operating table 12 which is capable of being tilted about a longitudinal axis indicated by 14. On the longitudinal sides of the operating table 12 a reference frame 20 is fastened above the operating table 12 via height-adjustable lateral supports 16, 18. Similar height-adjustable lateral supports are arranged further down towards the rear side of the operating table 12 (not discernible in FIG. 1). The reference frame 20 is configured for fastening a material-abrading tool, for example a milling cutter, that is needed during the intervertebral-disc operation, and for positioning this tool exactly in relation to the vertebral column.

Along the longitudinal sides of the operating table 12 a fluoroscope 22 is indicated which comprises an X-ray source 24 arranged on one longitudinal side of the operating table 12 and an image recorder 26 arranged on the opposite longitudinal side. The coverage area of the fluoroscope 22 is indicated by dotted lines 28. The fluoroscope 22 as such does not have to be aligned.

Rigidly fastened to the reference frame 20 is an alignment element 30 which extends into the coverage area of the fluoroscope 22.

Indicated by 32 is a patient who is lying on his back on the operating table 12; the outline of his head is indicated by 34. A vertebra 36 which delimits an intervertebral-disc compartment of the patient 32, into which an intervertebral-disc prosthesis is to be surgically inserted, has a vertebral plane indicated by 38 which normally extends parallel to the back of the patient 32 and therefore also parallel to the reclining surface of the operating table 12. Occasionally, however, the vertebral plane 38 is twisted by a few degrees about the longitudinal axis of the vertebral column. The tilt angle α is indicated in FIG. 1 relative to a horizontal plane 40.

The twisting of the vertebra 36 (and possibly of further neighboring vertebrae) shown in FIG. 1 may occur as a result of anatomical changes to the vertebral column of the patient 32. However, such a twisting may also arise only during the surgical intervention. In the course of preparing a ventral access to the intervertebral-disc compartment it may happen - as a result of the displacement of surrounding tissue, for example - that the vertebra 36 is twisted about the longitudinal axis of the vertebral column, and the vertebral plane 38 consequently tilts relative to the horizontal plane 40.

If in the case of a vertebra that has been twisted in such a manner an intervertebral-disc prosthesis were now to be inserted vertically from above into the intervertebral-disc compartment which has been exposed with the aid of the material-abrading tool, the intervertebral-disc prosthesis would not be positioned at the anatomically correct place. As a consequence of this, incorrect loadings and, associated therewith, complaints with respect to the vertebral column may occur.

The system 10 permits the material-abrading tool to be aligned correctly in relation to the twisted vertebra 36:

To this end, the operating table 12 is tilted about the tilt axis 14 until the vertebral plane 38 extends perpendicular to the image plane of the image recorded in the picture at number 26. In this rotary position of the vertebra 36 the transverse process 42 of the vertebra 36 pointing towards the X-ray source 24 overlaps the transverse process 44 pointing towards the image recorder 26, so that the latter transverse process is more or less completely overlapped by the anterior transverse process 42.

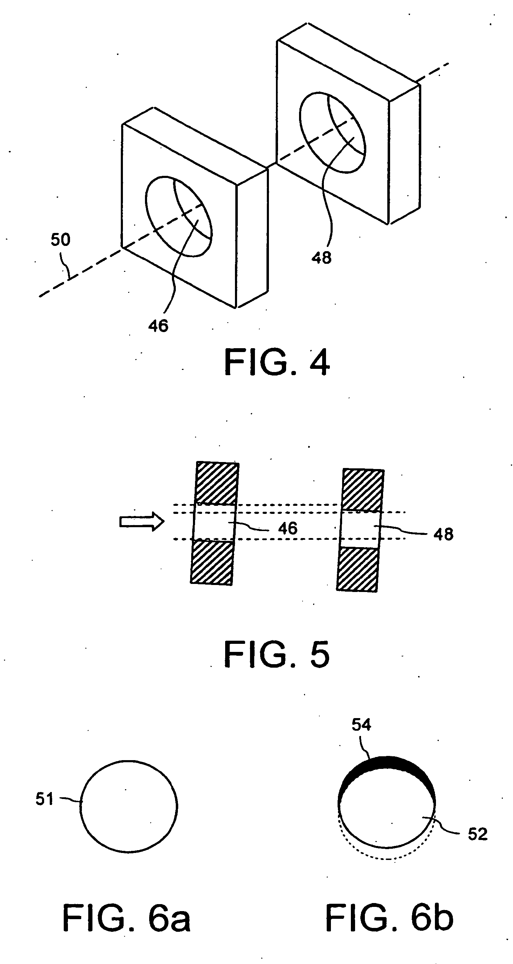

The state after the tilting of the operating table 12 is shown in FIG. 2. The vertebral plane 38 is now located in a defined position with respect to the fluoroscope 22 or the image that has been recorded thereby. As already mentioned, this defined position is distinguished in the present exemplary embodiment by the fact that the two transverse processes 42, 44 are aligned with one another, so that substantially only the transverse process 42 pointing towards the X-ray source 24 can be discerned in the recorded image. Since the reference frame 20 was firstly tilted together with the operating table 12 via the lateral supports 16, 18, the intervertebral-disc prosthesis would still be inserted incorrectly if a material-abrading tool were to be mounted on the now inclined reference frame 20. For this reason, in a second step the reference frame 20 is tilted in such a way that it is likewise transferred into a defined position with respect to the fluoroscope 22. For this purpose, in the exemplary embodiment shown, an alignment element 30 which is rigidly connected to the reference frame 20 comprises on its end a U-shaped bracket, through the shanks of which there extend two coaxial bores 46, 48. This is shown in a simplified manner in the perspective representation of FIG. 4. The alignment element 30 is arranged in such a way that the bores extend into the coverage area 28 of the fluoroscope 22. With the aid of the length-adjustable lateral supports 16, 18 the reference frame 20 is now tilted about an axis parallel to the tilt axis 14 in such a way that an axis of symmetry 50 extending through the bores 46, 48 extends perpendicular to the image plane of the image recorded by the fluoroscope 22. In the image this state can be discerned from the fact that the bores 46, 48 are imaged as a single circular disc 51, as shown in FIG. 6a. If the X-rays generated by the X-ray source 24 do not pass through the two bores 46, 48 parallel to the axis of symmetry 50 but inclined relative to it, no circular disc appears in the image. Instead, a segment 52 will be observed having the shape of disk which is reduced by a crescent-shaped shadow region 54 (see FIG. 6b). This shape arises for the reason that some of the X-rays that have passed through the first bore 46 cannot pass through the second bore 48, on account of their inclination relative to the axis of symmetry 50.

The reference frame 20 is now adjusted by adjustment of the lateral supports 16, 18 until the shadow region 54 disappears. The reference frame 20 is now mounted in a defined position with respect to the fluoroscope 22. Since the X-rays extend both parallel to the vertebral plane 38 and parallel to the plane of symmetry 50 of the bores 46, 48 of the alignment element 30, the vertebral plane 38 is now in a known angular position with respect to the reference frame 20.

Of course, for the alignment of the tool it is most expedient if the reference plane defined by the reference frame 20 extends parallel to the vertebral plane 38. This can be obtained in straightforward manner by the alignment element 30 being arranged exactly at a right angle relative to the reference frame 20.

The reference frame 20 now offers a reference system for the subsequent surgical steps. In FIG. 3 a material-abrading tool 56 with a milling head 58 is illustrated which has been aligned in relation to the reference frame 20 with the aid of devices that are known as such in the art and are therefore not represented in any detail. The tool 56 can now be moved in the transverse direction on the reference frame 20 via a slide or similar, in order to expose the intervertebral-disc compartment for the accommodation of the intervertebral-disc prosthesis.

In this connection it is also to be noted that in the case of the exemplary embodiment described above the X-rays do not necessarily have to pass through the bores 46, 48 of the alignment element 30 and through the vertebra 36 parallel to the horizontal plane 40. Such an exact alignment of the fluoroscope 22 would also be relatively difficult for practical reasons. Since both the vertebral plane 38 and the reference frame are aligned with respect to the fluoroscope, the absolute positioning of the fluoroscope 22 is irrelevant for the relative position between the reference frame 20 and the vertebral plane 38. For the correct positioning of the intervertebral-disc prosthesis, however, all that matters is that the reference frame is correctly aligned in relation to the vertebral plane 38.

## Claims

1. System for aligning a material-abrading tool (56, 58) relative to an intervertebral-disc compartment, comprising:
a) an operating table (12) having a longitudinal axis (14),
b) an image-recording device (22) for recording lateral images of the intervertebral-disc compartment,
c) a reference frame (20),
- which is arranged above the operating table (12),
- on which a material-abrading tool (56, 58) is capable of being fastened in a defined relative position with respect to the reference frame (20), and
d) an alignment element (30) which is rigidly connected to the reference frame (20) and is arranged such that it is detectable, together with the intervertebral-disc compartment, by the image-recording device (22),
**characterized in that**
- the operating table (12) is configured for being tilted about the longitudinal axis (14), and **in that**
- the reference frame (20) is configured for being tilted about a tilt axis which runs parallel to the longitudinal axis (14) of the operating table (12).

2. System according to Claim 1, **characterized in that** the image of the alignment element (30) detected by the image-recording device (22) corresponds to a predetermined pattern (51) if the reference frame (20) is located in a defined position with respect to the image-recording device (22).

3. System according to Claim 2, **characterized in that** the alignment element (30) includes at least one opening (46, 48).

## Patentansprüche

1. System zur Ausrichtung eines materialabtragenden Werkzeugs (56, 58) relativ zu einem Bandscheibenfach, mit:
a) einem Operationstisch (12), der eine Längsachse (14) hat,
b) einer bildgebenden Einrichtung (22) zur Aufnahme seitlicher Bilder des Bandscheibenfachs,
c) einen Referenzierungsrahmen (20),
- der oberhalb des Operationstisches (12) angeordnet ist,
- auf dem ein materialabtragendes Werkzeug (56, 58) in einer definierten Relativposition bezüglich des Referenzierungsrahmens (20) befestigbar ist, und
d) einem Ausrichtelement (30), das fest mit dem Referenzierungsrahmen (20) verbunden und derart angeordnet ist, dass es gemeinsam mit dem Bandscheibenfach von der bildgebenden Einrichtung (22) erfassbar ist,
**dadurch gekennzeichnet, dass**
- der Operationstisch (12) dazu eingerichtet ist, um die Längsachse (14) verkippt zu werden, und dass
- der Referenzierungsrahmen (20) dazu eingerichtet ist, um eine Kippachse verkippt zu werden, die parallel zur Längsachse (14) des Operationstisches (12) verläuft.

2. System nach Anspruch 1, **dadurch gekennzeichnet, dass** das von der bildgebenden Einrichtung (22) erfasste Bild des Ausrichtelements (30) einem vorgegebenen Muster (51) entspricht, wenn der Referenzierungsrahmen (20) sich in einer definierten Stellung bezüglich der bildgebenden Einrichtung (22) befindet.

3. System nach Anspruch 2, **dadurch gekennzeichnet, dass** das Ausrichtelement (30) wenigstens eine Öffnung (46, 48) aufweist.

## Revendications

1. Système destiné à aligner un outil d'abrasion de matériau (56, 58) par rapport à un logement intervertébral, comprenant :
a) une table d'opération (12) ayant un axe longitudinal (14),
b) un dispositif d'enregistrement d'images (22) destiné à enregistrer des images latérales du logement intervertébral,
c) une structure de référence (20),
- qui est disposée au-dessus de la table d'opération (12),
- sur laquelle un outil d'abrasion de matériau (56, 58) est en mesure d'être fixé dans une position relative définie par rapport à la structure de référence (20), et
d) un élément d'alignement (30) qui est relié de façon rigide à la structure de référence (20) et est disposé de telle sorte qu'il puisse être détecté, avec le logement intervertébral, par le dispositif d'enregistrement d'images (22), **caractérisé en ce que**
- la table d'opération (12) est configurée pour être inclinée autour de l'axe longitudinal (14), et **en ce que**
- la structure de référence (20) est configurée pour être inclinée autour d'un axe d'inclinaison qui s'étend parallèlement à l'axe longitudinal (14) de la table d'opération (12).

2. Système selon la revendication 1, **caractérisé en ce que** l'image de l'élément d'alignement (30) détecté par le dispositif d'enregistrement d'images (22) correspond à un motif prédéterminé (51) si la structure de référence (20) est située dans une position définie par rapport au dispostif d'enregistrement d'images (22).

3. Système selon la revendication 2, **caractérisé en ce que** l'élément d'alignement (30) comprend au moins une ouverture (46, 48).
